# EUROPEAN PATENT SPECIFICATION

(11) **EP 0 512 729 B1**
(45) Date of publication and mention of the grant of the patent: **26.07.1995**
(21) Application number: 92303792.3
(22) Date of filing: 27.04.1992
(51) Int. Cl.: A61B 17/22, A61B 17/28

(54) **Surgical instrument and its manufacturing method**
Chirurgisches Instrument und dessen Herstellungsverfahren
Instrument chirurgical et méthode de fabrication

(30) Priority: 03.05.1991 US 695222
(43) Date of publication of application: 11.11.1992
(73) Proprietor: VANCE PRODUCTS INCORPORATED d/b/a COOK UROLOGICAL INCORPORATED, Spencer Indiana 47460 (US)
(72) Inventor: Denson, Cynthia E., Aurora, Colorado 80014 (US); James, Benjamin F., Bloomington, Indiana 47403 (US)
(74) Representative: Johnston, Kenneth Graham

(56) References cited:
- EP-A- 0 160 870
- DE-A- 3 709 706

## Description

This invention relates to surgical instruments and their method of manufacturing.

A number of percutaneous surgical instruments are presently available for removing calculi from, for example, the urinary system. These surgical instruments typically include a wire basket extending from the distal end thereof for capturing and retaining calculi therein. However, these wire baskets are susceptible to causing trauma to surrounding tissue particularly when utilized in other percutaneous procedures such as in percutaneous gynaecological procedures. These gynaecological procedures typically involve grasping and manipulating soft tissue. The use of wire baskets for manipulating ovaries and other soft organs can easily result in undesired trauma. These resilient wire baskets are commonly collapsed and inserted into an introducer sheath and extended therefrom to assume their preformed shape when positioned at the treatment site. Alternatively, longitudinal slits are made about the distal end of a plastic tube and a control wire is inserted in the lumen thereof and attached to the distal end for opening and closing the longitudinal strips in the tubing formed as a result of the slits. Although well-suited for capturing urinary system calculi, the control wire of these plastic baskets prevents easy entry of soft tissue such as ovaries into the basket. Furthermore, the control wire can easily cause trauma to the soft tissue. The longitudinal plastic strips of the basket are usually of a high durometer for maintaining capture of a calculus and, as a result, can also easily cause trauma to the soft tissue.

Examples of surgical instruments are disclosed in U.S. Patent No.4,611,594, U.S. Patent No.2,556,783, and U.S. Patent No.4,807,626, which latter patent constitutes the prior art over which the independent claims 1 and 9 are characterized and discloses a basket which is expandable by tensioning a control line. Unfortunately, the control line limits the effective volume within the chamber. As a result, retaining an object in the chamber interferes with the tensioning and releasing of the control line. Another problem with this device is that force must be applied to both open the basket and maintain closure of the basket when calculi are contained therein and being extracted.

According to the present invention there is provided a surgical instrument as defined in claim 1 and a manufacturing method for such an instrument as defined in claim 9.

In an embodiment of the invention, the surgical instrument has a pliable plastic material basket having a collapsible, bulbous-shape formed about the distal end thereof for atraumatically capturing and manipulating soft tissue or an organ such as an ovary in delicate gynaecological endoscopic surgical procedures. The surgical instrument is insertable through a minimally invasive surgical access sheath for manipulating the soft tissue. The instrument comprises an elongated member which preferably has a longitudinal passageway extending therein. The basket is positioned about the distal end of the elongated member and includes a plurality of strips of pliable plastic material positioned about the distal end of the elongated member. The basket can be part of the elongated member when in tubular form. The strips of the basket are preformed into a bulbous shape and are collapsible for positioning in an introducer sheath.

The pliable plastic surgical instrument further includes an introducer sheath having a longitudinal passageway extending therethrough for positioning the basket therein in a collapsed state therein and introducing the basket into a cavity through the access sheath. When introduced into the cavity, the plastic basket automatically expands to resume its bulbous shape without any outside assistance such as a control line. Soft tissue such as an ovary is introduced into the basket between the strips and captured therein for subsequent manipulation. The basket may be advantageously further opened by applying a force to a small portion of the elongated member extending from about the distal end of the basket and elongated member. The preformed bulbous shape and soft material of the basket advantageously minimize trauma to the captured and manipulated tissue as well as surrounding tissue. When the soft tissue is captured in the basket, the proximal end of the basket is partially retracted into the introducer sheath to securely position the tissue within the basket.

The instrument further includes a collar about the proximal end of the introducer sheath and a longitudinally adjustably positionable clamp positioned around the proximal end of the elongated member for engaging the collar and maintaining the soft tissue within the partially retracted basket.

The basket is comprised of a plurality of longitudinal strips positioned about or at the distal end of the elongated member and comprises, for example, a soft polyurethane plastic material for minimizing trauma to soft tissue. The introducer sheath is formed of a polytetrafluoroethylene material for easily sliding the sheath and collapsed basket through the access sheath. The number of longitudinal strips is preferably four for securely grasping and manipulating the organ. However, three strips may be easily provided for engaging extremely large soft tissue organs. The basket may also be easily opened by pushing the distal end thereof with another instrument or other soft tissue for further opening and spreading apart the longitudinal strips.

### Brief description of the drawings

FIG.1 depicts an illustrative surgical instrument of the present invention having a pliable plastic material basket in a preformed open position;
FIG.2 depicts the instrument of FIG.1 with the basket in a collapsed state positioned in the introducer sheath;
FIG.3 depicts the instrument of FIG.1 with the basket of the instrument being extended from the introducer sheath after being introduced in a body cavity through an access sheath;
FIG.4 depicts the extended basket of FIG.3 being further opened for capturing a soft tissue organ; and
FIG.5 depicts the extended basket of FIG.4 partially retracted into the distal end of the introducer sheath for manipulating the soft tissue organ.

### Detailed description

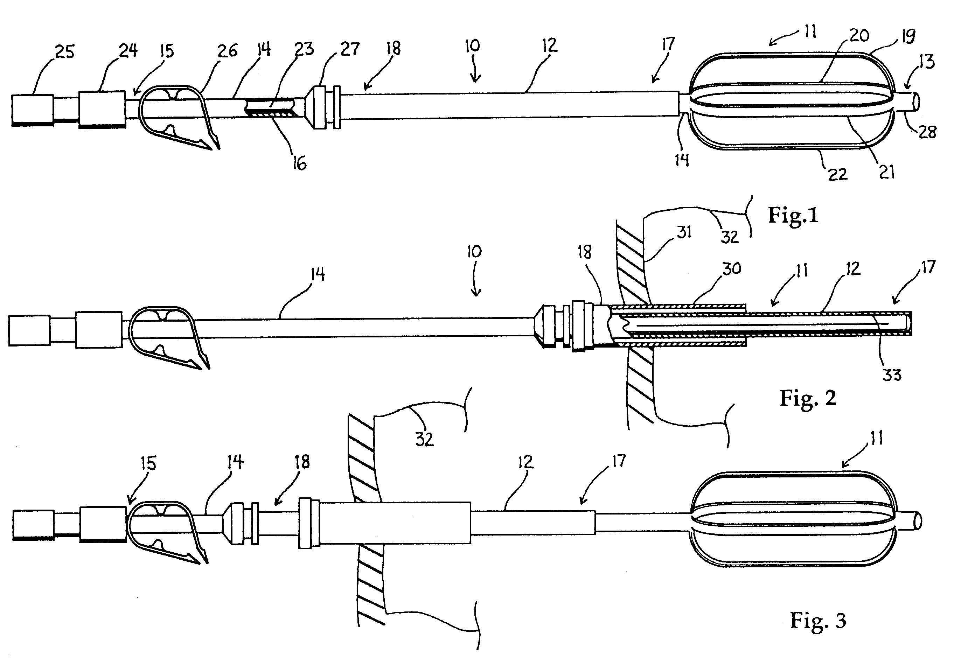

Depicted in FIG.1 is a preferred embodiment of an illustrative endoscopic surgical instrument 10 having a preformed pliable plastic material basket 11 which is collapsible within introducer sheath 12pp for insertion through a minimally invasive access sheath into a cavity of a patient. When introduced into the cavity of the patient, the basket is extended from the distal end of the introducer sheath to assume a preformed bulbous shape. The preformed basket is positioned about distal end 13 of elongated member tube 14 of pliable plastic material such as polyurethane. The elongated member includes distal end 13, proximal end 15, and longitudinal passageway (16) extending therethrough. For percutaneous insertion through an access sheath into the patient's body cavity, the elongated member tube is positioned within the longitudinal passageway of introducer sheath 12 and extends from distal end 17 and proximal end 18. When extended from the distal end of the introducer sheath, preformed basket 11 assumes a preformed bulbous shape formed from a plurality of longitudinal strips 19-22 of the pliable plastic material about the distal end of the elongated member. The preformed bulbous shape is formed by placing the longitudinal strips around a form and applying heat such as steam to the polyurethane material. Each of the strips has been pre-treated in such a way that the characteristics of the strips themselves have been altered. When fully expanded in the preformed shape, the basket includes a highly elongated chamber within the strips for surrounding soft tissue or an organ such as an ovary. The pliable plastic material strips atraumatically capture the soft tissue organ for subsequent manipulation when the proximal end of the basket is partially retracted into the introducer sheath. When captured within the basket, the soft tissue organ may be easily and atraumatically manipulated or repositioned for more direct visualization or convenient access with other endoscopic surgical instruments.

Elongated member 14 is formed from a pliable plastic material, preferably tubular, and of approximately 50cms. in length with a 12 French outside diameter (0.156") and an inside diameter of 0.254cms. (0.1"). A 30 cms. long stainless steel stylet rod 23 having a 0.173cms. ((0.068") diameter is inserted into passageway 16 about proximal end 15 of the elongated member tubing to stiffen the pliable material tubing for controlling introduction of the tubing into the proximal end of the introducer sheath. A commercially available proximal end connector 24, is fixedly positioned about the proximal end of the elongated member tubing through which the stylet rod is inserted and fixedly positioned with proximal end cap 25. The proximal end cap is a pin vise having a connector portion mating with proximal end connector 24 which is commercially available from Sabin Corporation, Bloomington, Indiana, or alternatively is moulded on to the proximal end of the stylet rod. Adhesive is applied to the proximal end cap and connector to fixedly position the two components together.

The surgical instrument further includes a releasable clamp 26 that is longitudinally slideable and adjustable along the length of the elongated member about the proximal end thereof. Releasable clamp 26 is commercially available, for example, from Halkey Medical of St. Petersburg, Florida. The clamp is slideable along the length of the proximal end of the elongated member to fixedly position the proximal end of the basket within the distal end of the introducer sheath. Introducer sheath includes a commercially available stop 27 such as a modified Molnar disk available from Vesta Corporation of Glendale, Wisconsin, positioned about proximal end 18. The stop and releasable clamp engage each other to prevent distal longitudinal withdrawal of the basket with the soft tissue therein from the distal end of the introducer sheath.

Introducer sheath is comprised of a polytetrafluoroethylene material tube approximately 33cms. in length and having an outside diameter of 13 French (0.194") and an inside diameter of 0.442cm. (0.174"). The introducer sheath is made of a rigid material and has a slick outside surface for sliding easily into and through the minimally invasive access sheath.

Preformed basket 11 in an open position is approximately 5.2cms. in length with a 3.4cms. diameter. The basket is formed into a bulbous, ellipsoidal shape for further expansion and entry of soft tissue organs therein. A short portion 28 of approximately 5mms. in length elongated member tubing extends from the distal end of the basket to the distal end of the tube. This short portion of tubing is formed by heating, moulding, and buffing the distal end of the tubing to a rounded surface for engagement against other tissue in the cavity or another surgical instrument. When the short portion is engaged, the strips of the bulbous-shaped basket are spread further apart to receive large soft tissue organs such as an ovary therein. Alternatively, basket 11 may be formed of a soft plastic material and attached at the distal end of a stiffer material elongated member. As a result, the stiffer elongated member need not have the stylet rod positioned at the proximal end thereof and need not be tubular.

The method of manipulating soft tissue such as an ovary with minimally invasive surgical instrument 10 includes introducing access sheath 30 percutaneously through abdominal wall 31 into cavity 32 as depicted in FIG.2. Basket 11 in a collapsed state is positioned into longitudinal passageway 33 of introducer sheath 12 as shown. The introducer sheath is introduced through the access sheath and into body cavity 32. When basket 11 is positioned in the introducer sheath, the stylet rod, which is proximally positioned in elongated member 14, extends into introducer sheath passageway 33 about proximal end 18. This may be helpful in allowing the physician to extend the basket from distal end 17 of the introducer sheath into the body cavity to assume its preformed bulbous shape.

Depicted in FIG.3 is basket 11 in its preformed bulbous shape extended into body cavity 32 from distal end 17 of introducer sheath 12. The basket is extended from the distal end of the introducer sheath by the physician pushing proximal end 15 of elongated member tube 14 into proximal end 18 of the introducer sheath, thereby extending the basket from the distal end of the introducer sheath.

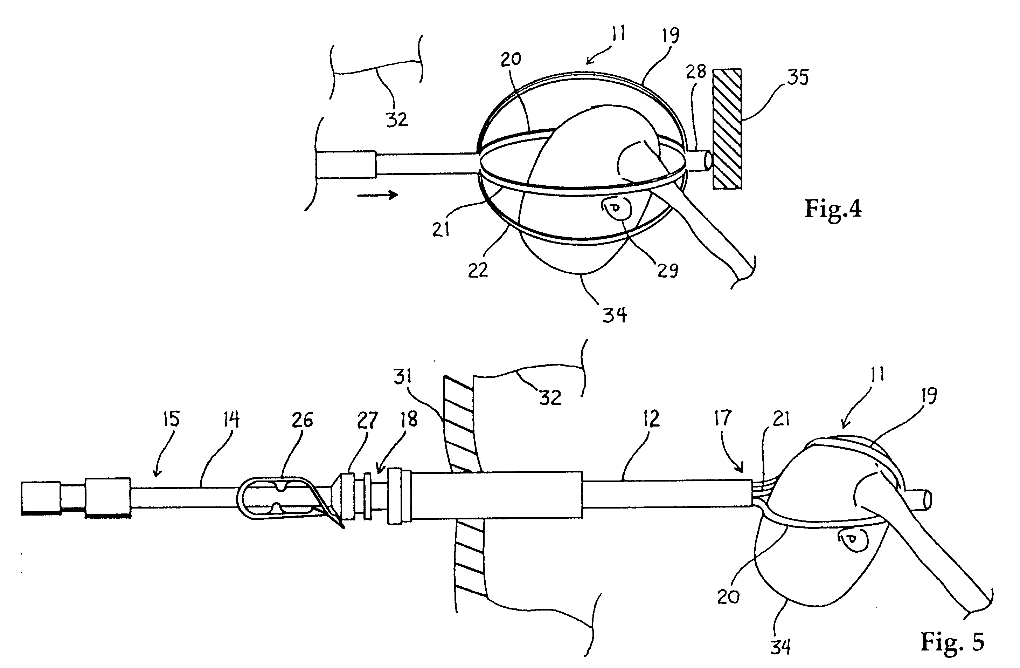

Depicted in FIG.4 is basket 11 in body cavity 32 with strips 19-22 being further spread apart for capturing ovary 34 therein. Distal portion 28 further spreads the strips of the bulbous, ellipsoidal shaped basket further apart by engaging soft tissue 35 within the cavity or another endoscopic instrument inserted in the cavity. Ovary 34 is positioned within the basket, and the longitudinal strips allowed to collapse around the ovary. As a result, the ovary is easily manipulated to allow the physician to better visualize irregularities such as cyst 29 thereon.

Depicted in FIG.5 is ovary 34 within basket 11 with longitudinal strips 19-21 (22 not shown) partially retracted into distal end 17 of introducer sheath 12. The partially retracted basket permits the longitudinal strips of the basket to securely and atraumatically engage ovary 34. To fixedly position the relative longitudinal position of the basket with the ovary therein with respect to the introducer sheath, slideable clamp 26 is slid along proximal end 15 of elongated member tube 14 to engage stop 27 about proximal end 18 of the introducer sheath. This allows the physician to manipulate the surgical instrument with the ovary fixedly positioned within the basket thereof.

The particular preformed bulbous shape of the basket may be elongated or shortened or extended into a larger spherical shape to capture and manipulate various size soft tissue organs atraumatically. Furthermore, the basket may be utilized to capture and retrieve other tougher tissue such as fibrous tumours, myomas, muscle, etc., that may be encountered in minimally invasive surgical procedures.

## Claims

1. A surgical instrument (10) insertable through an access sheath (30) and for manipulating soft tissue in a body cavity (32) during minimally invasive surgery, said instrument comprising an elongated member (14) having proximal (15) and distal (13) ends, and with a basket (11) formed at the said distal end from a plurality of strips (19 to 22) of plastic material, the strips being in an unstressed condition when the basket is in an expanded condition, the basket being collapsible for its insertion through the access sheath, characterised in that the strips have been heated in a preformed condition to form a preformed bulbous shaped basket, the preformation being such that the strips automatically expand when the basket exits the access sheath and resumes the expanded or preformed condition.

2. The instrument of claim 1, further characterised by an introducer sheath (12) having a longitudinal passageway (33) extending therethrough, said basket in a collapsed state and said elongated member being insertable through said passageway of said introducer sheath.

3. The instrument of claim 2, characterised in that the elongated member has an internal passageway, and in that a stylet (23) is positioned about said proximal end of said elongated member and extends into the passageway of said introducer sheath when said basket in said collapsed state is positioned in said introducer sheath.

4. The instrument of claim 2 or 3, further characterised by a collar (27) fixedly positioned about said proximal end of said introducer sheath.

5. The instrument of claim 4, further characterised by a clamp (26) adjustably positionable longitudinally about said proximal end of said elongated member for engaging said collar, and optionally comprising a connector (24) fixedly positioning said stylet about said proximal end of said elongated member.

6. The instrument of claim 1, characterised in that said elongated member includes a portion (28) extending between said distal end and said basket for engaging tissue and enlarging said basket when longitudinal pressure is applied to said portion.

7. The instrument of any one preceding claim, characterised in that the basket includes three or four longitudinal strips.

8. The instrument of any one preceding claim, characterised in that the plastic material is soft polyurethane plastic material.

9. A method of making a surgical instrument (10) insertable through an access sheath in order to manipulate soft tissue in a body cavity during minimally invasive surgery, said instrument comprising an elongated member (14) having proximal (15) and distal (17) ends, and with a basket (11) formed at the said distal end from a plurality of longitudinal strips (19 to 22) of pliable plastic material, the basket being collapsible for insertion through the access sheath, characterised in that the method comprises the steps of placing the longitudinal strips around a bulbous shaped form, and of heating the strips so that they assume a preformed shape corresponding to the shape of a preformed bulbous shaped basket, such preformation causing the strips to automatically expand the basket when it exits the access sheath, and to cause the basket to assume an unstressed condition.

## Patentansprüche

1. Chirurgisches Instrument (10), das durch einen Zugangsschaft (30) einführbar ist und mit dem während minimalinvasiver Chirurgie Weichteile in einer Körperhöhle (32) gehandhabt werden können, wobei jenes Instrument ein längliches Element (14) mit einem proximalen (15) und einem distalen (13) Ende und mit einem Korb (11) umfaßt, der an jenem distalen Ende aus einer Mehrzahl von Streifen (19 bis 22) aus Kunststoffmaterial gebildet ist, die in einem entspannten Zustand sind, wenn der Korb in einem gespreizten Zustand ist, wobei der Korb für sein Einführen durch den Zugangsschaft zusammenklappbar ist, dadurch gekennzeichnet, daß die Streifen in einem vorgeformten Zustand zur Bildung eines vorgeformten knollenförmigen Korbes erhitzt worden sind, wobei es sich um eine derartige Vorformung handelt, daß sich die Streifen automatisch spreizen, wenn der Korb den Zugangsschaft verläßt und den gespreizten oder vorgeformten Zustand einnimmt.

2. Instrument nach Anspruch 1, weiterhin gekennzeichnet durch eine Einführhülse (12) mit einem sich durch sie hindurch erstreckenden länglichen Durchgang (33), wobei jener Korb im zusammengeklappten Zustand und jenes längliche Element durch jenen Durchgang jener Einführhülse eingeführt werden können.

3. Instrument nach Anspruch 2, dadurch gekennzeichnet, daß das längliche Element einen inneren Durchgang aufweist und daß um jenes proximale Ende jenes länglichen Elements ein Mandrin (23) angeordnet ist, der sich in den Durchgang jener Einführhülse erstreckt, wenn jener Korb in jenem zusammengeklappten Zustand in jener Einführhülse angeordnet ist.

4. Instrument nach Anspruch 2 oder 3, weiterhin gekennzeichnet durch einen fest um jenes proximale Ende jener Einführhülse angeordneten Bund (27).

5. Instrument nach Anspruch 4, weiterhin gekennzeichnet durch eine Klemme (26), die länglich um jenes proximale Ende jenes länglichen Elements zur Ineingriffnahme jenes Bundes verstellbar angeordnet werden kann, wobei jenes Instrument gegebenenfalls einen Verbinder (24) umfaßt, der jenen Mandrin fest um jenes proximale Ende jenes länglichen Elements anordnet.

6. Instrument nach Anspruch 1, dadurch gekennzeichnet, daß jenes längliche Element einen sich zwischen jenem distalen Ende und jenem Korb erstreckenden Abschnitt (28) einschließt, der Gewebe in Eingriff nimmt und jenen Korb vergrößert, wenn jener Abschnitt mit Längsdruck beaufschlagt wird.

7. Instrument nach einem der vorhergehenden Ansprüche, dadurch gekennzeichnet, daß der Korb drei oder vier längliche Streifen einschließt.

8. Instrument nach einem der vorhergehenden Ansprüche, dadurch gekennzeichnet, daß es sich bei dem Kunststoffmaterial um weiches Polyurethan-Kunststoffmaterial handelt.

9. Verfahren zur Herstellung eines chirurgischen Instrumentes (10), das durch einen Zugangsschaft einführbar ist, um während minimalinvasiver Chirurgie Weichteile in einer Körperhöhle zu handhaben, wobei jenes Instrument ein längliches Element (14) mit einem proximalen (15) und einem distalen (13) Ende und mit einem Korb (11) umfaßt, der an jenem distalen Ende aus einer Mehrzahl von länglichen Streifen (19 bis 22) aus biegsamem Kunststoffmaterial gebildet ist und der zur Einführung durch den Zugangsschaft zusammenklappbar ist, dadurch gekennzeichnet, daß das Verfahren folgende Schritte umfaßt: die länglichen Streifen werden um eine knollenförmige Form gelegt und erhitzt, so daß sie eine der Gestalt eines vorgeformten knollenförmigen Korbes entsprechende vorgeformte Gestalt annehmen, wobei die Streifen durch eine derartige Vorformung automatisch den Korb spreizen, wenn er den Zugangsschaft verläßt, und ihn dazu veranlassen, einen entspannten Zustand einzunehmen.

## Revendications

1. Instrument chirurgical (10) insérable à travers un fourreau d'accès (30) et pour manipuler des tissus mous dans une cavité (32) du corps durant une intervention chirurgicale faiblement envahissante, ledit instrument comprenant une pièce allongée (14) ayant des extrémités proximale (15) et distale (13), et avec un panier (11) formé à ladite extrémité distale à partir d'une pluralité de rubans (19 à 22) en matériau plastique, les rubans étant dans une condition dénuée de contrainte quand le panier est dans une condition renflée, le panier étant repliable pour son insertion à travers le fourreau d'accès, caractérisé en ce que les rubans ont été chauffés dans une condition préformée pour former un panier préformé de forme bulbeuse, la préformation étant telle que les rubans se détendent automatiquement vers l'extérieur quand le panier sort du fourreau d'accès et reprend la condition renflée ou préformée.

2. Instrument de la revendication 1, caractérisé de plus par un fourreau d'introduction (12) ayant un passage longitudinal (33) s'étendant à travers ce dernier, ledit panier dans un état replié et ladite pièce allongée étant insérables à travers ledit passage dudit fourreau d'introduction.

3. Instrument de la revendication 2, caractérisé en ce que la pièce allongée comporte un passage interne, et en ce qu'un stylet (23) est positionné au niveau de ladite extrémité proximale de ladite pièce allongée et s'étend dans le passage dudit fourreau d'introduction quand ledit panier dans ledit état replié est positionné dans ledit fourreau d'introduction.

4. Instrument de la revendication 2 ou 3, caractérisé de plus par un collet (27) positionné fixement autour de ladite extrémité proximale dudit fourreau d'introduction.

5. Instrument de la revendication 4, caractérisé de plus par une pince (26) pouvant être positionnée longitudinalement par réglage autour de ladite extrémité proximale de ladite pièce allongée pour engager ledit collet, et comprenant optionnellement un raccord (24) positionnant fixement ledit stylet au niveau de ladite extrémité proximale de ladite pièce allongée.

6. Instrument de la revendication 1, caractérisé en ce que ladite pièce allongée inclut une partie (28) s'étendant entre ladite extrémité distale et ledit panier pour venir en contact avec les tissus et pour agrandir ledit panier quand une pression longitudinale est appliquée à ladite partie.

7. Instrument de l'une quelconque des revendications précédentes, caractérisé en ce que le panier inclut trois ou quatre rubans longitudinaux.

8. Instrument de l'une quelconque des revendications précédentes, caractérisé en ce que le matériau plastique est un matériau plastique en polyuréthane mou.

9. Méthode de fabrication d'un instrument chirurgical (10) insérable à travers un fourreau d'accès de manière à manipuler des tissus mous dans une cavité du corps durant une intervention chirurgicale faiblement envahissante, ledit instrument comprenant une pièce allongée (14) ayant des extrémités proximale (15) et distale (17), et avec un panier (11) formé à ladite extrémité distale à partir d'une pluralité de rubans longitudinaux (19 à 22) en matériau plastique souple, le panier étant repliable pour insertion à travers le fourreau d'accès, caractérisé en ce que la méthode comprend les étapes consistant à placer les rubans longitudinaux autour d'un objet de forme bulbeuse, et à chauffer les rubans de sorte qu'ils prennent une forme préformée correspondant à la forme d'un panier préformé de forme bulbeuse, une telle préformation incitant les rubans à détendre automatiquement le panier vers l'extérieur quand il sort du fourreau d'accès, et à inciter le panier à adopter une condition dénuée de contrainte.
